(19) Europäisches Patentamt
European Patent Office
Office européen des brevets

(11) Veröffentlichungsnummer: **0 004 897**
**B1**

(12) # EUROPÄISCHE PATENTSCHRIFT

(45) Veröffentlichungstag der Patentschrift: **18.03.81**

(21) Anmeldenummer: **79100991.3**

(22) Anmeldetag: **31.03.79**

(51) Int. Cl.³: **C 07 D 249/04,** C 07 D 249/18, C 07 D 403/04, C 07 D 403/10, C 07 D 403/12, C 07 D 405/14, C 07 D 411/04, C 07 D 401/04, C 07 D 413/04, C 07 D 417/04, C 07 D 413/10 // C07D261/12, C07D413/12, C07D417/12, C07D405/04

(54) **Verfahren zur Herstellung von in 2-Stellung substituierten Triazolen-1,2,3.**

(30) Priorität: **13.04.78 DE 2815956**

(43) Veröffentlichungstag der Anmeldung: **31.10.79 Patentblatt 79/22**

(45) Bekanntmachung des Hinweises auf die Patenterteilung: **18.03.81 Patentblatt 81/11**

(84) Benannte Vertragsstaaten: **CH DE FR GB IT**

(56) Entgegenhaltungen:
**US-A-3 790 491**
**ADVANCES IN HETEROCYCLIC CHEMISTRY vol. 16, 1974, New York London T.L. Gilchrist und G.E. Gymer, »1,2,3 triazolos« Seiten 33—85**
**HETEROCYCLIC COMPOUNDS, vol. 7 1961, New York London J.H. Boyer: »Monocyclic Triazoles and Benzotriazols«, Seiten 385—460**

(73) Patentinhaber: **BAYER AG, Zentralbereich Patente, Marken und Lizenzen, D-5090 Leverkusen 1, Bayerwerk (DE)**

(72) Erfinder: **Knupfer, Hans, Dr., Katharinental 5, D-5060 Bergisch-Gladbach 2 (DE)**
Erfinder: **Schellhammer, Carl-Wolfgang, Dr., Katharinental 26, D-5060 Bergisch-Gladbach 2 (DE)**

**EP 0 004 897 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

Verfahren zur Herstellung von in 2-Stellung substituierten Triazolen-1,2,3

Die Erfindung betrifft ein Verfahren zur Herstellung von in 2-Stellung substituierten Triazolen-1,2,3 der Formel

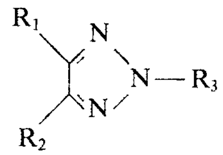

(I)

in der

$R_1$ Wasserstoff oder $R_2$,

$R_2$ einen gegebenenfalls substituierten Alkyl-, Aralkyl-, Cycloalkyl- oder Alkenylrest, einen aromatisch-carbocyclischen oder aromatisch-heterocyclischen Rest, eine Carboxy-, Sulfo-, Ester- oder Amidgruppe bedeuten oder — gemeinsam mit $R_1$ — unter Einschluß der Kohlenstoffatome des Triazolringes, einen carbo- oder heterocyclischen Ring bilden und

$R_3$ der Rest eines aromatisch-carbocyclischen oder aromatisch-heterocyclischen Systems ist.

Das Verfahren ist dadurch gekennzeichnet, daß man Verbindungen der Formel

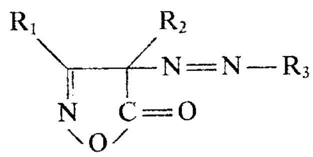

(II)

in der

$R_1$, $R_2$ und $R_3$ die oben angegebene Bedeutung haben, mit einer basischen Verbindung behandelt.

Die Reste $R_1$ und $R_2$ stehen vorzugsweise für $C_1-C_{18}$-Alkyl, das beispielsweise durch —OH, Halogen wie Cl, Br und F, Amino, Reste primärer und sekundärer Amine, Alkoxy, insbesondere $C_1-C_4$-Alkoxy, Cyan, Carboxy, Carbalkoxy, gegebenenfalls N-substituiertes Carbamoyl, Alkyl- oder Arylsulfonyl, gegebenenfalls N-substituiertes Sulfamoyl, substituiert sein kann; Cycloalkyl insbesondere Cyclohexyl und Cyclopentyl; Alkenyl insbesondere Vinyl, Allyl und Propenyl; Aryl wie Phenyl, das durch Halogen, Alkyl, Alkoxy, Nitro, Cyan, Phenyl, Carboxy, Sulfo, Carbalkoxy, gegebenenfalls N-substituiertes Carbamoyl oder gegebenenfalls N-substituiertes Sulfamoyl, Sulfonsäureester, Acylreste wie Alkyl- oder Arylcarbonyl, substituiert sein kann; gegebenenfalls substituiertes Naphthyl; Aralkyl wie gegebenenfalls substituiertes Benzyl; heterocyclische Reste wie Pyridyl, Thienyl; Aralkyl, insbesondere gegebenenfalls substituierte Benzyl- oder Phenylethylreste.

$R_1$ und $R_2$ können auch zusammen mit den Kohlenstoffatomen des Triazolringes, an die sie gebunden sind, einen heterocyclischen oder carbocyclischen, beispielsweise 5- oder 6-gliedrigen carbocyclischen Ring, bilden.

Geeignete Substituenten $R_3$ sind neben aromatisch-carbocyclischen Resten wie gegebenenfalls substituiertes Phenyl oder gegebenenfalls substituiertes Naphthyl, Reste aromatisch-heterocyclischer Systeme wie Pyridyl-, Cumarinyl-7, das vorzugsweise in 3-Stellung weitere Substituenten, vorzugsweise Aryl wie gegebenenfalls substituiertes Phenyl und gegebenenfalls substituiertes Naphthyl, oder Alkoxycarbonyl oder aromatisch-heterocyclische Reste wie Triazolyl wie z. B. 1.2.4-Triazolyl-1, 1.2.3-Triazolyl-1 oder 1.2.3-Triazolyl-2, wobei die Triazolylreste weitere Substituenten oder annellierte Ringe tragen können, aufweisen kann. Beispiele für derartig substituierte Triazolylreste sind 3- und/oder 5-Alkyl-, Aryl-, Aralkyl-1.2.4-triazolyl-1; 4- und/oder 5-Alkyl, Aryl- oder Aralkyl-1.2.3-triazolyl-1 bzw. -triazolyl-2, wobei die Reste in 4- und 5-Stellung zusammen auch die restlichen Glieder eines annellierten carbocyclischen Ringes wie beispielsweise eines Benzol- oder Naphthalinringes bilden können; Pyrazolyl wie Pyrazolyl-1 sowie substituiertes Parazolyl-1, wobei als Substituenten insbesondere Halogen vorzugsweise Chlor, Alkyl, Aryl, Aralkyl in Frage kommen, und wobei sich diese Substituenten vorzugsweise in 4-Stellung des Pyrazolyl-1-Restes befinden; Thienyl-2; Carbostyrilyl-7; in 2-Stellung substituiertes Benztriazolyl-5 wie 2-Styrylbenztriazolyl-5; in 5-Stellung substituiertes 1.2.4-Thiadiazolyl-3 wie 5-Phenyl-1.2.4-thiadiazolyl; 2-Methyl-benzoxazolyl-5 oder -6 und -benzthiazolyl-5 oder 6.

Geeignete basische Verbindungen sind insbesondere Stickstoffbasen wie Amine insbesondere tertiäre Amine und bistertiäre Diamine wie Trialkylamine und Dialkyl-arylamine, die weitersubstituiert sein können. Besonders genannt seien Trialkylamine mit 1 bis 6 C-Atomen je Alkylrest, die beispielsweise durch Hydroxy oder Äthoxy substituiert sein können. Als geeignete basische Verbindungen seien weiterhin Alkali- und Erdalkalicarbonate genannt.

Die basischen Verbindungen werden dabei in Mengen von etwa 0,01—10 Mol bezogen auf 1 Mol der Verbindung (II) eingesetzt.

Die Umsetzungstemperatur hängt von der Art der eingesetzten Verbindung (II) ab und liegt im allgemeinen zwischen etwa 20° C und etwa 150° C, vorzugsweise zwischen 60—120° C.

Die Umsetzung wird zweckmäßigerweise in einem Verdünnungsmittel durchgeführt. Geeignete Verdünnungsmittel sind beispielsweise die obengenannten tertiären Amine, weiterhin Alkohole, insbesondere sekundäre aliphatische Alkohole wie Isopropanol, Isobutanol oder Aminoalkohole wie 1-Dimethylamino-propanol.

Die Ausgangsverbindungen (II) sind teilweise literaturbekannt; beispielsweise die Verbindung der Formel

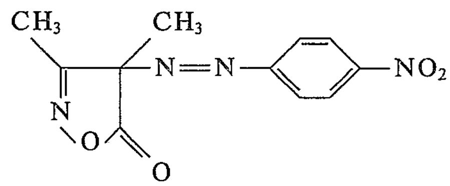

(III)

aus Chem. Pharm. Bull. (Tokyo) 12, 1021 (1969) und die Verbindung

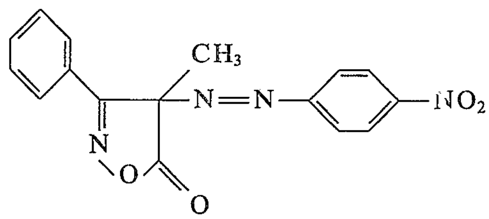

(IV)

aus J. Chem. Soc. 1965, 5414.

Die Verbindung (IV) erhält man durch Umsetzung von p-Nitrophenyldiazoniumchlorid mit 3-Phenyl-4-methyl-isoxazolin-5-on.

Nach diesem Verfahren erhält man auch die Verbindungen (II) der vorliegenden Anmeldung aus den entsprechenden Diazoniumsalzen

$$[R_3—N_2]^{\oplus} X^{\ominus} \qquad (V)$$

worin

$X^{\ominus}$ ein Anion, insbesondere $Cl^{\ominus}$ oder $SO_4^{2\ominus}$ darstellt und den Isoxazolin-5-on-Verbindungen der Formel

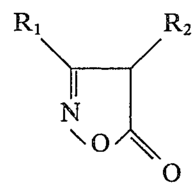

(VI)

Geeignete Diazoniumsalze (V) sind insbesondere solche, die sich von den folgenden Aminen ableiten: Anilin; durch eine oder mehrere Alkylgruppen mit vorzugsweise 1—4 C-Atomen, Alkenylgruppen, Aralkylengruppen, Nitrogruppen, Halogenatome, z. B. F, Cl oder Br, Cyangruppen, Carboxygruppen bzw. deren Derivate, Sulfonsäuregruppen bzw. deren Derivate, Alkyl- bzw. Aryl-sulfonylgruppen, Alkoxy- bzw. Aryloxy-gruppen, Aralkylgruppen, Arylgruppen, Aminogruppen, Alkylaminogruppen, Dialkylaminogruppen und/oder Acylaminogruppen substituierte Aniline; entsprechend substituierte Naphthylamine-1 oder -2; Phenanthrylamine, Anthranylamine; Pyrenylamine; 4,4'-Diaminostilben, das gegebenenfalls weitere Substituenten insbesondere Sulfo, Sulfamoyl, Carboxy, Alkoxycarbonyl, oder Cyan trägt; Aminopyridine, 2-Aminobenzothiazol, 3-Amino-5-phenyl-thiadiazol-1.2.4; 5-Aminobenzotriazol; 4-Amino-naphthalimide, die am Imidstickstoff durch gegebenenfalls weitersubstituierte Alkyl-, Aralkyl-, Alkenyl- oder Arylreste substituiert sind.

Geeignete Amine zur Herstellung der Diazoniumsalze (V) sind ferner:

5-Amino-2-styryl-benzoxazole, die gegebenenfalls im Styrylrest weiter substituiert sind;
6-Amino-2-styryl-benzoxazole, die gegebenenfalls im Styrylrest weiter substituiert sind;
5-Amino-benzothiazole, die in 2-Stellung durch Alkyl- oder gegebenenfalls weiter substituierte Styrylreste substituiert sind;
6-Amino-2-styryl-benzothiazole, die gegebenenfalls im Styrylrest weiter substituiert sind;
5-Amino-2-styryl-benzotriazole, die gegebenenfalls im Styrylrest weiter substituiert sind;

2-p-Amino-styryl-benzoxazole, die gegebenenfalls im Benzokern des Benzoxazols weiter substituiert sind;

2-p-Amino-styryl-benzothiazole, die gegebenenfalls im Benzokern des Benzothiazols weiter substituiert sind;

2-p-Amino-styryl-benzotriazole, die gegebenenfalls im Benzokern des Benzotriazols weiter substituiert sind;

2-p-Amino-styryl-naphthothiazole;

2-p-Amino-styryl-naphthotriazole;

6-Amino-benzofurane, die in 2-Stellung gegebenenfalls weiter substituiert sind;

2-p-Aminophenyl-benzofuran

7-Amino-3-phenyl-phenoxathiazin-2,2-dioxid

3-Aryl-7-amino-carbostyrile;

3-(p-Aminophenyl)-carbostyril

7-Amino-cumarincarbonsäure-3-alkylester;

7-Amino-cumarin, das gegebenenfalls weiter substituiert ist, vorzugsweise in 3-Stellung und vorzugsweise durch Aryl wie gegebenenfalls substituiertes Phenyl und gegebenenfalls substituiertes Naphthyl oder aromatisch-heterocyclische Reste wie Triazolyl beispielsweise 1.2.4-Triazolyl-1, 1.2.3-Triazolyl-1, 1.2.3-Triazolyl-2, wobei diese Reste weitere Substituenten bzw. annellierte Ringe aufweisen können. Beispiele für derartige substituierte Triazolylreste sind 3- und/oder 5-Alkyl-, -Aryl-, -Aralkyl-1.2.4-triazolyl-1, 4- und/oder 5-Alkyl-, -Aryl- oder -Aralkyl-1.2.3-triazolyl-1 bzw. -triazolyl-2, wobei die Reste in 4- und 5-Stellung zusammen auch die restlichen Glieder eines annellierten carbocyclischen Ringes wie beispielsweise eines gegebenenfalls substituierten Benzolringes oder gegebenenfalls substituierten Naphthalinringes bilden können.

Pyrazolyl wie Pyrazolyl-1, sowie substituiertes Pyrazolyl wobei als Substituenten insbesondere Halogen vorzugsweise Chlor, Alkyl, Aryl, Aralkyl in Frage kommen, wobei diese Substituenten vorzugsweise in 4-Stellung sitzen und Thienyl-2.

Geeignete Isoxazolin-5-one sind beispielsweise

4-Methyl-i (i = Isoxazolinon-5), 3,4-Dimethyl-i, 4-β-Hydroxyäthyl-i, 3-Methyl-4-äthyl-i, 3-Methyl-4-β-hydroxyäthyl-i, 4-γ-Aminopropyl-i, 3-Cyclopropyl-4-methyl-i, 3,4-Diäthyl-i, 3-Methyl-4-isopropyl-i, 3-Methyl-4-tert.-butyl-i, 3-Propyl-4-äthyl-i, 3-Methyl-4-äthyl-i, 3-Methyl-4-(2-methyl-2-hydroxypropyl)-i, 4-Phenyl-i, 3-Methyl-4-cyclopentyl-i, 3-Methyl-4-phenyl-i, 3-Phenyl-4-methyl-i, 3-Butyl-4-propyl-i, 3-p-Bromphenyl-4-β-hydroxyäthyl-i, 3-p-Chlorphenyl-4-β-hydroxyäthyl-i, 3-p-Nitrophenyl-4-β-hydroxyäthyl-i, 3-Äthyl-4-phenyl-i, 3-Phenyl-4-äthyl-i, 3-Methyl-4-benzyl-i, 3-p-Tolyl-4-β-hydroxyäthyl-i, 3-tert.-Butyl-4-phenyl-i, 3-Methyl-4-(1-phenylpropyl)-i, 3-Phenyl-4-(4-pyridyl)-i, 3-Phenyl-4-(3-pyridyl)-i, 3-(3.4.5-Trimethoxyphenyl)-4-β-hydroxyäthyl-i, 3-Phenyl-4-(2-chlorphenyl)-i, 3,4-Diphenyl-i, 3-Methyl-4-benzyl-i, 3-p-Methoxyphenyl-4-methyl-i, 3-p-Chlorphenyl-4-methyl-i, 3-Biphenylyl-4-methyl-i, 3,4-Tetramethylen-i, 3,4-Trimethylen-i.

Die genannten Isoxazoline sind zum größten Teil literaturbekannt. Soweit dies nicht der Fall ist, lassen sie sich analog den bekannten aus den entsprechenden Ausgangsverbindungen erhalten.

Im Vergleich zu der aus der US-PS 3 790 491 bekannten Triazolsynthese liefert das erfindungsgemäße Verfahren die Triazole (I), welche z. B. wertvolle optische Aufheller sind, in wesentlich besseren Ausbeuten und höherer Reinheit, da die zur Gewinnung der Ausgangsmaterialien durchzuführende Kupplung von (V) auf (VI) glatter verläuft als die entsprechende Kupplung der Diazoniumsalze auf das Benzylmethylketon gemäß Beispiel 1 jener Publikation.

Schließlich weisen die in »Advances in Heterocyclic Chemistry, Vol. 16, p. 61 (1964)« und in »Heterocyclic Compounds, Vol. 7, p. 400—401 (1961)« beschriebenen Isoxazol-Umlagerungen gegenüber der anspruchsgemäßen Methode den Nachteil auf, daß sie explosionsartig (vgl. die auf Seite 401 zitierten Berichte, 61, 11 [1928]) verlaufen und damit für Synthesen im technischen Maßstab praktisch unbrauchbar sind. Im übrigen sind alle vorstehend abgehandelten Verfahren des Standes der Technik weniger universell anwendbar, sondern auf spezielle Systeme (z. B. Ketone) beschränkt.

## Herstellungsbeispiele für die Azoverbindungen

A. 23,7 g (0,1 Mol) 7-Amino-3-phenyl-cumarin werden bei höchstens 30°C in 240 ml konzentrierter Schwefelsäure gelöst. Man kühlt die Lösung auf 10°C ab und diazotiert mit 32 g (0,106 Mol) Nitrosylschwefelsäure. Nach 2 Stunden trägt man auf Eis aus und saugt das Diazoniumsalz ab. 17,5 g (0,1 Mol) 3-Phenyl-4-methyl-isoxazolin-5-on werden in 150 ml Eisessig gelöst. Man gibt 30 g kristallines Natriumacetat hinzu und trägt bei 16°C oder darunter die oben hergestellte Paste des Diazoniumsalzes ein. Am nächsten Tage wird die gelbe Azoverbindung abgesaugt, mit Wasser gewaschen und bei 50°C getrocknet. Ausbeute 41 g. Eine aus Benzol/Leichtbenzin umgelöste Probe schmilzt bei 134°C unter Zersetzung.

B. 18 g (0,1 Mol) 5-Amino-3-phenyl-1,2,4-thiadiazol werden in 300 ml 85%iger Phosphorsäure gelöst. Nach Stehen über Nacht versetzt man mit 100 ml Eisessig und diazotiert in 3 Stunden mit 32 g (0,106 Mol) Nitrosylschwefelsäure bei 0°C bis −2°C. Nach 25 Stunden Stehen bei −5°C tropft man eine Lösung von 19,3 g (0,11 Mol) 3-Phenyl-4-methyl-isoxazolin-5-on in 150 ml Eisessig in 25 Minuten hinzu und rührt 1 Stunde nach. Man trägt die Mischung auf Eiswasser aus, saugt die gelbe Azoverbindung ab und trocknet sie nach dem Waschen mit Wasser. Ausbeute 35,5 g. Eine aus Benzol umgelöste Probe schmilzt bei 118°C unter Zersetzung.

C. 14,3 g (0,054 Mol) 7-Amino-1-äthyl-3-phenyl-carbostyril werden in 150 ml heißem Eisessig gelöst. Man versetzt die Lösung mit 20 ml konzentrierter Salzsäure und kühlt sie auf 0°C ab. Bei 0—5°C wird mit einer Lösung von 3,8 g (0,055 Mol) Natriumnitrit in 10 ml Wasser diazotiert. Zu der Diazoniumsalzlösung gibt man nach 2 Stunden eine Lösung von 15 g (0,059 Mol) 3-p-Biphenylyl-4-methyl-isoxazolin-5-on in 150 ml Eisessig. Die gelbe Azoverbindung wird am nächsten Tage abgesaugt, mit Wasser gewaschen und getrocknet. Ausbeute 27,5 g, F. 111—112°C unter Zersetzung.

Beispiele für Umlagerungen zur v-Triazolverbindung

A. 26,5 g 3-Phenyl-4-methyl-isoxazolin-5-on-4-azo-4-nitrobenzol (Rohware) werden in 120 ml Dichlormethan gelöst; die Lösung wird mit Tonsil geklärt und dann zu einer 70°C warmen Mischung aus 200 ml Triäthylamin und 200 ml Isopropanol getropft. Dabei wird fortlaufend das Methylenchlorid abdestilliert. Man kocht die Mischung dann noch 3 Stunden rückfließend, kühlt auf +10°C ab und saugt das 2-p-Nitrophenyl-4-methyl-5-phenyl-1,2,3-triazol ab. Ausbeute 7,1 g, F. 160—164°C.

B. 27,7 g 3-Phenyl-4-benzyl-isoxazolin-5-on-4-azo-7-(3-p-chlorphenyl)-cumarin werden in 175 ml Xylol suspendiert und zu 150 ml auf 110°C erwärmten 1-Dimethylamino-propanol-(2) in 5 Minuten zugegeben. Man kocht die Mischung dann noch eine halbe Stunde rückfließend, konzentriert den Kolbeninhalt durch Anlegen von Vakuum auf 75 ml und versetzt mit 200 ml Methanol. Das abgeschiedene Produkt wird abgesaugt und getrocknet. Ausbeute 17,5 g, F. 198—200°C. Nach dem Umlösen aus Glykolmonomethyl-ätheracetat erhält man 12,6 g schwach gelbe Kristalle von F. 199—202°C.

Analog den vorstehenden Beispielen werden die in der Tabelle aufgeführten Verbindungen hergestellt.

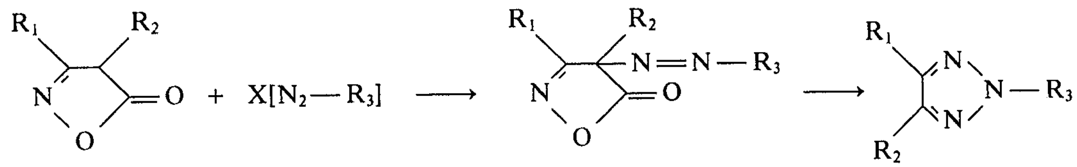

| Nr. | $R_1$ | $R_2$ | $R_3$ | Azoverbindung Verfahren | Azoverbindung F (°C)$_z$ | Triazolverbindung Verfahren | Triazolverbindung F (°C) |
|---|---|---|---|---|---|---|---|
| 1 | $-CH_3$ | $-CH_2-C_6H_5$ | 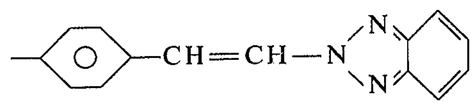 | C | 134 | B | 187–189 |
| 2 | $-C_6H_5$ | $-CH_3$ | desgl. | C | 132 | B | 200–203 |
| 3 | $-C_6H_5$ | $-CH_3$ | 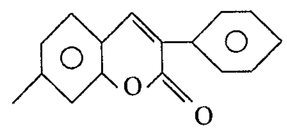 | A | 124 | A, B | 164–165 |
| 4 | $-CH_3$ | $-CH_2-C_6H_5$ | desgl. | A | 133 | B | 170–171 |
| 5 | $-CH_3$ | $-C_6H_5$ | desgl. | A | 97–102 | A | 164–165 |
| 6 | $-C_6H_5$ | $-CH_2-C_6H_5$ | desgl. | A | 118–119 | B | 173–175 |
| 7 | 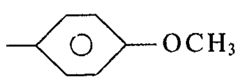 | $-CH_3$ | desgl. | A | 103–111 | B | 176–179 |
| 8 | 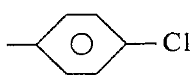 | $-CH_3$ | desgl. | A | 125–126 | B | 181–184 |
| 9 | $-C_6H_5$ | $-CH_2-C_6H_5$ | desgl. | B | 128–130 | B | 199–202 |
| 10 | $-C_6H_5$ | $-C_6H_5$ | desgl. | B | 104–106 | B | 247–248 |

(Fortsetzung)

| Nr. | $R_1$ | $R_2$ | $R_3$ | Azoverbindung Verfahren | Azoverbindung F (°C)$_z$ | Triazolverbindung Verfahren | Triazolverbindung F (°C) |
|---|---|---|---|---|---|---|---|
| 11 | $—C_6H_5$ | $—CH_3$ | 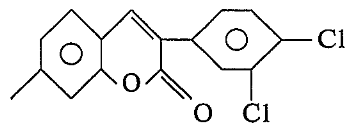 | B | 113–114 | B | 246–248 |
| 12 | $—C_6H_5$ | $—C_6H_5$ | desgl. | B | 81 | B | 200–203 |
| 13 | $—C_6H_5$ | $—CH_3$ | 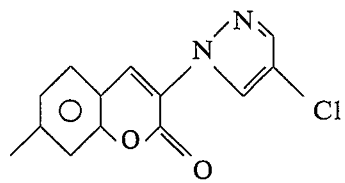 | A | 124 | A | 239–241 |
| 14 | $—CH_3$ | $—CH_2—C_6H_5$ | desgl. | A | 132 | A, B | 201–202 |
| 15 | 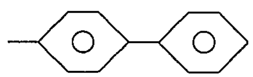 | $—CH_3$ | desgl. | A | 126–128 | A | 287–288 |
| 16 | $—C_6H_5$ | $—CH_2—C_6H_5$ | desgl. | A | 139–140 | B | 172–175 |
| 17 | 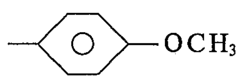 | $—CH_3$ | desgl. | A | 125–127 | B | 251–253 |
| 18 | 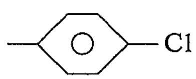 | $—CH_3$ | desgl. | A | 114–115 | B | 254–257 |
| 19 | 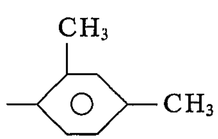 | $—CH_3$ | desgl. | A | 63 | A | 207–209 |

(Fortsetzung)

| Nr. | $R_1$ | $R_2$ | $R_3$ | Azoverbindung Verfahren | F (°C), | Triazolverbindung Verfahren | F (°C) |
|---|---|---|---|---|---|---|---|
| 20 | 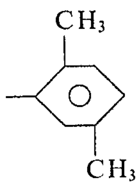 | $-CH_3$ | 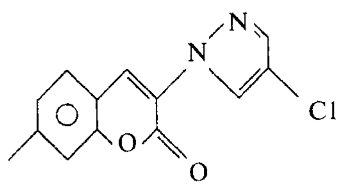 | A | 63 | A | 205–206 |
| 21 | $-C_6H_5$ | $-CH_3$ | 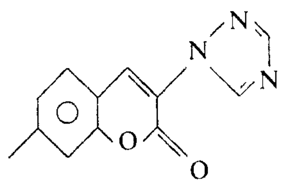 | C | 123 | A | 236–237 |
| 22 | $-CH_3$ | $-CH_2-C_6H_5$ | desgl. | C | – | A | 202–203 |
| 23 | $-C_6H_5$ | $-CH_2-C_6H_5$ | desgl. | C | 65–70 | B | 185–186 |
| 24 | 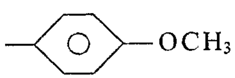 | $-CH_3$ | desgl. | A | 117–118 | B | 220–222 |
| 25 | 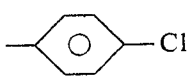 | $-CH_3$ | desgl. | A | 117–118 | B | 229–231 |
| 26 | $-CH_3$ | $-CH_2-C_6H_5$ | 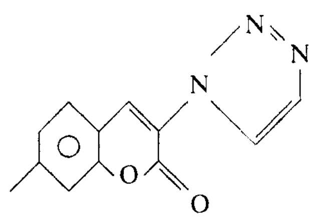 | C | 103–107 | A | 194–195 |

(Fortsetzung)

| Nr. | $R_1$ | $R_2$ | $R_3$ | Azoverbindung Verfahren | Azoverbindung F (°C)$_z$ | Triazolverbindung Verfahren | Triazolverbindung F (°C) |
|---|---|---|---|---|---|---|---|
| 27 | $—C_6H_5$ | $—CH_3$ | 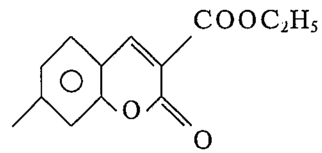 | C | 110 | B | 182 |
| 28 | $—C_6H_5$ | $—CH_2—C_6H_5$ | desgl. | C | 114–115 | B | 151–152 |
| 29 | $—C_6H_5$ | $—CH_2—C_6H_5$ | 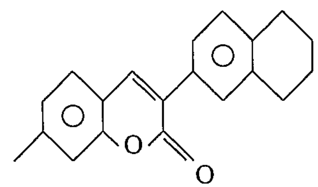 | C | 131–133 | B | 233–234,5 |
| 30 | $—C_6H_5$ | $—CH_3$ | 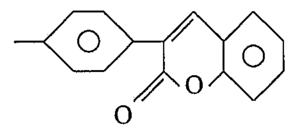 | C | 100 | A | 161–165 |
| 31 | $—C_6H_5$ | $—CH_3$ | 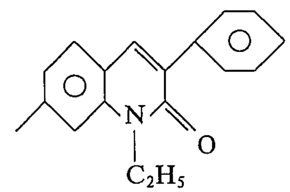 | C | 120 | A | 151–153 |
| 32 | 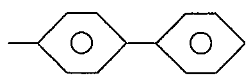 | $—CH_3$ | desgl. | C | 111–112 | A | 202–203 |

(Fortsetzung)

| Nr. | $R_1$ | $R_2$ | $R_3$ | Azoverbindung Verfahren | Azoverbindung F (°C)$_z$ | Triazolverbindung Verfahren | Triazolverbindung F (°C) |
|---|---|---|---|---|---|---|---|
| 33 | $-C_6H_5$ | $-CH_3$ | 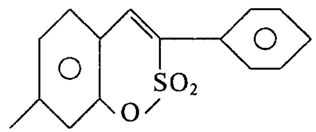 | C | 110 | B | 140–142 |
| 34 | $-C_6H_5$ | $-CH_3$ | 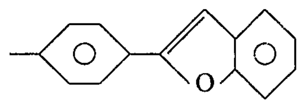 | C | 115–116 | B | 176–178 |
| 35 | $-C_6H_5$ | $-CH_3$ | 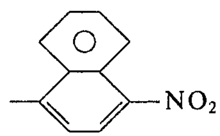 | C | 108 | A | 120–122 |
| 36 | $-C_6H_5$ | $-CH_3$ | 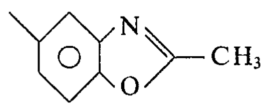 | C | – | A | 130–132 |
| 37 | $-C_6H_5$ | $-CH_3$ | 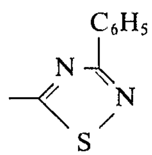 | B | 118 | A | 138–139 |
| 38 | $-C_6H_5$ | $-CH_3$ | 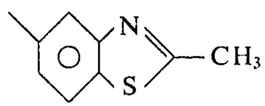 | C | – | A | 103–105 |
| 39 | $-C_6H_5$ | $-CH_3$ | 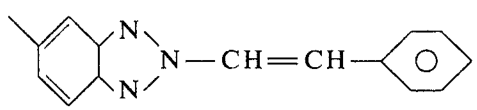 | C | 70 | B | 143–146 |

(Fortsetzung)

| Nr. | $R_1$ | $R_2$ | $R_3$ | Azoverbindung Verfahren | Azoverbindung F (°C)$_z$ | Triazolverbindung Verfahren | Triazolverbindung F (°C) |
|---|---|---|---|---|---|---|---|
| 40 | 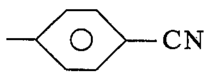 | $-CH_3$ | 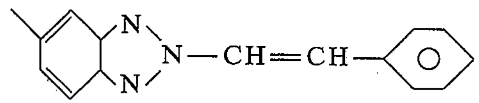 | C | 105–110 | B | 240–243 |
| 41 | $-CH_3$ | $-CH_2-C_6H_5$ | desgl. | C | 117–119 | B | 136–137 |
| 42 | $-C_6H_5$ | $-CH_3$ | 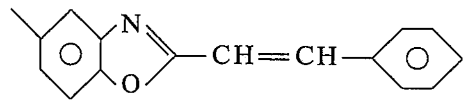 | C | 69 | A | 186–187 |
| 43 | $-C_6H_5$ | $-CH_3$ | 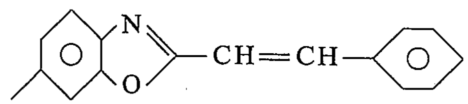 | C | 75–79 | A | 163–164,5 |
| 44 | $-C_6H_5$ | $-CH_3$ | 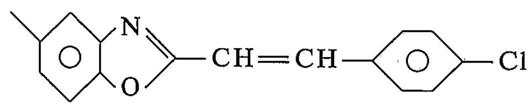 | C | 90–94 | A | 219–221 |
| 45 | $-C_6H_5$ | $-CH_3$ | desgl. | C | 114–116 | A | 196–197 |
| 46 | $-C_6H_5$ | $-CH_3$ | 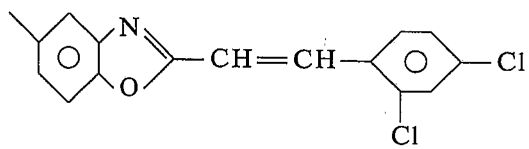 | C | 93–101 | A | 185 |
| 47 | $-C_6H_5$ | $-CH_3$ | 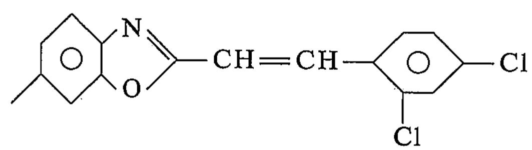 | C | 114–115 | A | 216–219 |

(Fortsetzung)

| Nr. | $R_1$ | $R_2$ | $R_3$ | Azoverbindung Verfahren | Azoverbindung F (°C)$_z$ | Triazolverbindung Verfahren | Triazolverbindung F (°C) |
|---|---|---|---|---|---|---|---|
| 48 | $-CH_3$ | $-CH_2-C_6H_5$ | 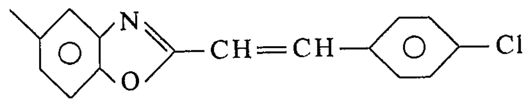 | C | 116–118 | B | 202 |
| 49 | $-CH_3$ | $-CH_2-C_6H_5$ | 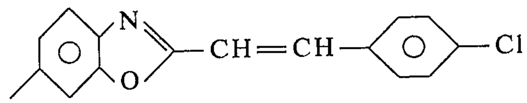 | C | 115–123 | A | 181–183 |
| 50 | $-C_6H_5$ | $-CH_2-C_6H_5$ | desgl. | C | 99–105 | B | 176–178 |
| 51 | $-CH_3$ | $-CH_2-C_6H_5$ | 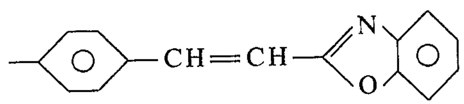 | C | 101–105 | A | 175–178 |
| 52 | $-C_6H_5$ | $-CH_3$ | 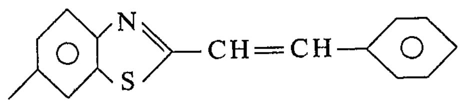 | C | 65–72 | A | 173–175 |
| 53 | $-C_6H_5$ | $-CH_3$ | 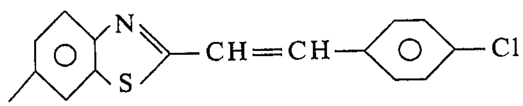 | C | 99–102 | A | 216–218 |
| 54 | $-CH_3$ | $-CH_2-C_6H_5$ | desgl. | C | 109–111 | A | 179–181 |
| 55 | $-C_6H_5$ | $-CH_3$ | 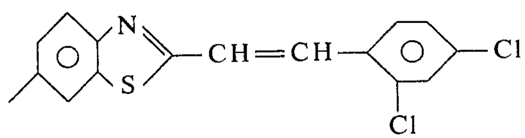 | C | 103–105 | A | 224–226 |

(Fortsetzung)

| Nr. | $R_1$ | $R_2$ | $R_3$ | Azoverbindung Verfahren | Azoverbindung F (°C)$_z$ | Triazolverbindung Verfahren | Triazolverbindung F (°C) |
|---|---|---|---|---|---|---|---|
| 56 | $-CH_3$ | $-CH_2-C_6H_5$ | 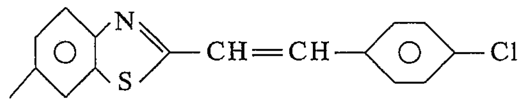 | C | 126 | A | 187–189 |
| 57 | 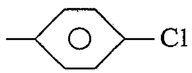 | $-CH_3$ | desgl. | C | 110–112 | B | 262–265 |
| 58 | 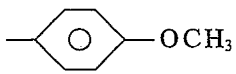 | $-CH_3$ | desgl. | C | 125–126 | B | 219–223 |
| 59 | $C_6H_5$ | $-CH_2-C_6H_5$ | desgl. | C | 102–108 | B | 190–192 |
| 60 | 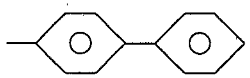 | $-CH_3$ | desgl. | C | 68–79 | B | 233–234 |
| 61 | 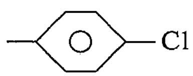 | $-CH_3$ | desgl. | C | 110–118 | B | 290–292 |
| 62 | $-C_6H_5$ | $-C_6H_5$ | 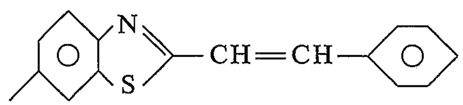 | C | 83–84 | B | 206–208 |
| 63 | $-C_6H_5$ | $-CH_3$ | 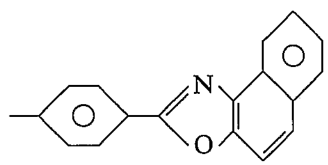 | C | 119–120 | A | 206–207 |
| 64 | 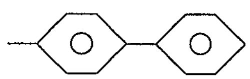 | $-CH_3$ | desgl. | C | 105 | B | 252–255 |

(Fortsetzung)

| Nr. | $R_1$ | $R_2$ | $R_3$ | Azoverbindung Verfahren | F (°C)z | Triazolverbindung Verfahren | F (°C) |
|---|---|---|---|---|---|---|---|
| 65 | $-C_6H_5$ | $-CH_3$ | 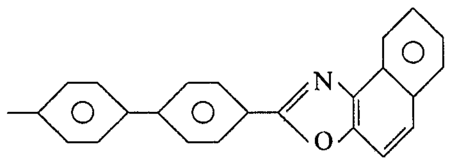 | C | 121 | B | 226–227 |
| 66 | $-C_6H_5$ | $-CH_2-C_6H_5$ | desgl. | C | 131–132 | B | 174–176 |
| 67 | 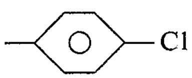Cl | $-CH_3$ | desgl. | C | 123–125 | B | 245–246 |
| 68 | $-C_6H_5$ | $-C_6H_5$ | desgl. | C | 99–101 | B | 238–241 |
| 69 | $-CH_3$ | $-CH_2-$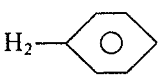 | desgl. | C | 124–125 | B | 235–236 |

**Patentansprüche**

1. Verfahren zur Herstellung von Verbindungen der Formel

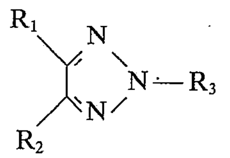

(I)

in der

$R_1$ Wasserstoff oder $R_2$,
$R_2$ einen gegebenenfalls substituierten Alkyl-, Aralkyl-, Cycloalkyl- oder Alkenylrest, einen aromatisch-carbocyclischen oder aromatisch-heterocyclischen Rest, eine Carboxy-, Sulfo-, Ester- oder Amidgruppe bedeuten oder — gemeinsam mit $R_1$ — unter Einschluß der Kohlenstoffatome des Triazolringes einen carbo- oder heterocyclischen Ring bilden und
$R_3$ der Rest eines aromatisch-carbocyclischen oder aromatisch-heterocyclischen Systems sind,

dadurch gekennzeichnet, daß man Verbindungen der Formel

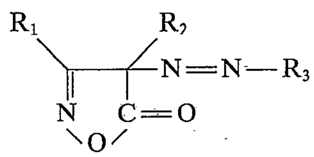

(II)

in der
$R_1$, $R_2$ und $R_3$ die oben angegebene Bedeutung haben,
gegebenenfalls in Gegenwart eines Verdünnungsmittels bei Temperaturen zwischen etwa 20° C bis etwa 150° C mit einer basischen Verbindung behandelt.

2. Verfahren gemäß Anspruch 1, dadurch gekennzeichnet, daß man als basische Verbindung ein tertiäres Amin verwendet.

**Claims**

1. Process for the preparation of compounds of the formula

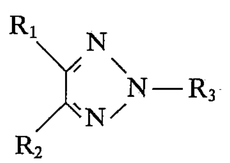

(I)

in which

$R_1$ denotes hydrogen or $R_2$,
$R_2$ denotes an optionally substituted alkyl, aralkyl, cycloalkyl or alkenyl radical, an aromatic-carbocyclic or aromatic-heterocyclic radical, a carboxy, sulpho, ester or amide group, or — together with $R_1$ — with the inclusion of the carbon atoms of the triazole ring, form a carbocyclic or heterocyclic ring and
$R_3$ is the radical of an aromatic-carbocyclic or aromatic-heterocyclic system,

characterised in that compounds of the formula

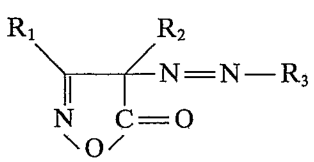

(II)

in which
$R_1$, $R_2$ and $R_3$ have the meaning indicated above, are treated with a basic compound, if appropriate in the presence of a diluent at temperatures of between about 20° C and about 150° C.

2. Process according to claim 1, characterised in that a tertiary amine is used as the basic compound.

## Revendications

1. Procédé pour la préparation de composés de formule

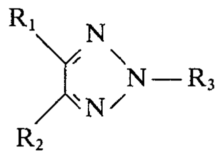

(I)

dans laquelle:

$R_1$ représente l'hydrogène ou $R_2$,
$R_2$ représente un reste alkyle, arylalkyle, cycloalkyle ou alcényle éventuellement substitué, un reste aromatique carbocyclique ou hétérocyclique, un groupe carboxy, sulfo, ester ou amide, ou bien $R_1$ et $R_2$ pris ensemble avec les atomes de carbone du noyau triazolique forment un noyau carbocyclique ou hétérocyclique, et
$R_3$ est le reste d'un système aromatique carbocyclique ou hétérocyclique,

caractérisé en ce que l'on fait réagir des composés de formule

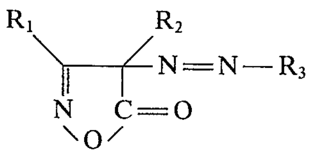

(II)

dans laquelle $R_1$, $R_2$ et $R_3$ sont tels que définis ci-dessus, avec un composé basique éventuellement en présence d'un agent diluant, à des températures comprises entre environ 20 et 150° C.

2. Procédé selon la revendication 1, caractérisé en ce que l'on utilise comme composé basique une amine tertiaire.